# EUROPEAN PATENT APPLICATION

(11) **EP 4 190 363 A1**
(43) Date of publication of application: **07.06.2023**
(21) Application number: 21850937.0
(22) Date of filing: 29.07.2021
(51) Int. Cl.: A61K 48/00, A61P 21/02, A61P 23/02, A61P 25/16, A61P 25/28, A61P 43/00, C12N 15/11, A61K 31/7125

(54) **MIR-96-5P INHIBITOR AND PHARMACEUTICAL COMPOSITION CONTAINING SAME**

(30) Priority: 29.07.2020 JP 2020128453
(71) Applicant: Teikyo University, Tokyo 173-8605 (JP)
(72) Inventor: NAKAKI Toshio, Tokyo 173-8605 (JP); AOYAMA Koji, Tokyo 173-8605 (JP); KINOSHITA Chisato, Tokyo 173-8605 (JP); MATSUMURA Nobuko, Tokyo 173-8605 (JP); UTSUMI Kazue, Tokyo 173-8605 (JP); SUGIYAMA Toru, Tokyo 173-8605 (JP); MORIYA Shun-suke, Tokyo 173-8605 (JP)
(74) Representative: Gille Hrabal Partnerschaftsgesellschaft mbB Patentanwälte
(86) International application number: PCT/JP2021/028004
(87) International publication number: WO 2022/025148

(57) **Abstract**

Provided a novel miR-96-5p inhibitor that is more effective than an antisense oligonucleotide against miR-96-5p, and a novel pharmaceutical composition comprising the same. The miR-96-5p inhibitor comprises a peptide nucleic acid moiety comprising a nucleotide sequence complementary to miR-96-5p.

## Description

### TECHNICAL FIELD

The present invention relates to an miR-96-5p inhibitor and a pharmaceutical composition containing the same.

### BACKGROUND ART

In neurodegenerative diseases such as Alzheimer's disease and Parkinson's disease, it is known that intraneuronal glutathione is reduced. Glutathione is a major antioxidant in the nervous system. The present inventors found that microRNA96-5p (miR-96-5p) is a key molecule in regulating intraneuronal glutathione concentration, and that miR-96-5p has the effect of suppressing glutathione levels, and furthermore, the inventors were able to demonstrate in live mice that the amount of intraneuronal glutathione increased by inhibiting the action of miR-96-5p in the brain (Non-patent literature 1). Furthermore, the present applicant has obtained a patent (Patent literature 1) relating to a pharmaceutical composition for prevention or treatment of neurodegenerative diseases containing an antisense oligonucleotide of human miR-96-5p as an active ingredient.

### CITATION LIST

### PATENT LITERATURE

[Patent literature 1] JP 6342288 B

### NON-PATENT LITERATURE

[Non-patent literature 1] Kinoshita, C. et al. Rhythmic oscillations of the microRNA miR-96-5p play a neuroprotective role by indirectly regulating glutathione levels. Nat. Commun. 5:3823 doi: 10.1038/ncomms4823 (2014)

### SUMMARY OF INVENTION

### TECHNICAL PROBLEM

A problem is to provide a novel miR-96-5p inhibitor that is more effective than an antisense oligonucleotide against miR-96-5p, which is a known miR-96-5p inhibitor, and a novel pharmaceutical composition comprising the same.

### SOLUTION TO PROBLEM

The problem can be solved by the following inventions:
[1] An miR-96-5p inhibitor comprising a peptide nucleic acid moiety comprising a nucleotide sequence complementary to miR-96-5p.
[2] The miR-96-5p inhibitor of [1], wherein a peptide moiety consisting of 1 to several amino acids is added to an N-terminus and/or a C-terminus of the peptide nucleic acid moiety.
[3] The miR-96-5p inhibitor of [1] or [2], wherein the peptide nucleic acid moiety consists only of a nucleotide sequence complementary to miR-96-5p.
[4] The miR-96-5p inhibitor of any one of [1] to [3], wherein the nucleotide sequence complementary to miR-96-5p is a nucleotide sequence of SEQ ID NO: 1.
[5] A pharmaceutical composition comprising the miR-96-5p inhibitor of any one of [1] to [4] as an active ingredient.
[6] The pharmaceutical composition of [5] for prevention or treatment of a neurodegenerative disease in which a decrease in intraneuronal glutathione is observed.
[7] The pharmaceutical composition of [5] or [6], wherein the neurodegenerative disease is Alzheimer's disease, Parkinson's disease, multiple system atrophy, or amyotrophic lateral sclerosis.

### ADVANTAGEOUS EFFECTS OF INVENTION

The miR-96-5p inhibitor of the present invention has a peptide nucleic acid backbone, and is not degraded by nucleases. Therefore, as a therapeutic agent for neurodegenerative diseases that are chronic diseases, the inhibitory effect of the PNA on miR-96-5p is expected to last longer than known antisense oligonucleotides. Therefore, since the number of administrations is less than that of known antisense oligonucleotides, it can be used advantageously.

### BRIEF DESCRIPTION OF THE DRAWINGS

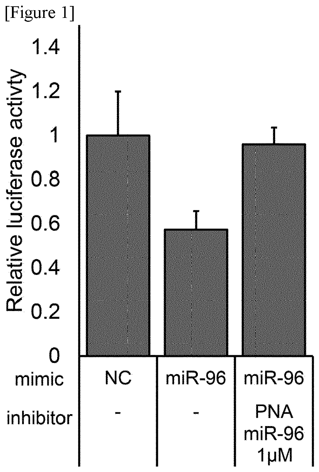

Fig. 1 is a graph showing the results of examining the miR-96-5p inhibitory effect of the miR-96-5p inhibitor of the present invention by luciferase reporter assay.

### DESCRIPTION OF EMBODIMENTS

### <<miR-96-5p inhibitor of the present invention>>

As long as the miR-96-5p inhibitor of the present invention (hereinafter also simply referred to as the inhibitor of the present invention) comprises at least a peptide nucleic acid moiety comprising a nucleotide sequence complementary to miR-96-5p, and has an inhibitory effect on miR-96-5p activity (hereinafter referred to as an miR-96-5p inhibitory effect), it can comprise an additional moiety that is covalently linked to the peptide nucleic acid moiety.

The inhibitor of the present invention can, for example, consist only of the peptide nucleic acid moiety, or can consist of the peptide nucleic acid moiety and the additional moiety.

It is known that miR-96-5p exists in various organisms, such as humans, pigs, mice, rats, and the like. It is not particularly limited to these organisms, but is preferably humans.

**[Table 1]**

| Species | ID | Sequence | SEQ ID NO: |
|---|---|---|---|
| Human | hsa-miR-96-5p | UUUGGCACUAGCACAUUUU UGCU | 2 |
| Pig | ssc-miR-96-5p | UUUGGCACUAGCACAUUUU UGCU | 2 |
| Mouse | mmu-miR-96-5p | UUUGGCACUAGCACAUUUU UGCU | 2 |
| Rat | rno-miR-96-5p | UUUGGCACUAGCACAUUUU UGCU | 2 |

The term "nucleotide sequence complementary to miR-96-5p" as used herein means a nucleotide sequence that exhibits the miR-96-5p inhibitory effect and is 90% or more, preferably 95% or more, and more preferably 100% complementary to the entire miR-96-5p sequence or a continuous partial sequence thereof. As the continuous partial sequence, for example, a sequence in which terminal 3 nucleotides (in the case of both ends, the sum of both ends) are deleted from the entire miR-96-5p sequence, preferably a sequence in which terminal 2 nucleotides are deleted, and more preferably a sequence in which a terminal 1 nucleotide is deleted, may be exemplified.

The term "peptide nucleic acid" as used herein means an artificially chemically synthesized nucleic acid analogue, which is also called PNA (Peptide Nucleic Acid). It has a structure in which the basic backbone of nucleic acid, which consists of pentose and phosphate, is replaced with an uncharged polyamide backbone with glycine as the unit. More particularly, it is a compound in which the sugar-phosphate backbone of nucleic acid is replaced with a backbone of N-(2-aminoethyl)glycine as the unit, and bases are linked by a methylene carbonyl bond. It binds to nucleic acids with complementary nucleotide sequences more specifically and strongly than DNA or RNA. On the other hand, since it is a chemically synthesized substance, it has the property that nucleic acid polymerases and nucleases do not act on it.

The "peptide nucleic acid moiety comprising a nucleotide sequence complementary to miR-96-5p" in the inhibitor of the present invention consists entirely of peptide nucleic acid, and its nucleotide sequence comprises a nucleotide sequence complementary to miR-96-5p. Therefore, the nucleotide sequence of the peptide nucleic acid moiety can consist only of a nucleotide sequence complementary to miR-96-5p, or can be a nucleotide sequence in which a nucleotide sequence(s) consisting of peptide nucleic acid is added to the N-terminus and/or the C-terminus of a nucleotide sequence complementary to miR-96-5p (it should have the miR-96-5p inhibitory effect).

In the peptide nucleic acid moiety, the nucleotide sequence(s) that is added to the N-terminus and/or the C-terminus of a nucleotide sequence complementary to miR-96-5p is not particularly limited, as long as the entire nucleotide sequence (i.e., a nucleotide sequence complementary to miR-96-5p in which the nucleotide sequence(s) is added to the N-terminus and/or the C-terminus) exhibits the miR-96-5p inhibitory effect. A nucleotide sequence in which the number of nucleotides (in the case of both ends, the sum of both ends) is, for example, 1 to several, preferably 1 to 6, more preferably 1 to 5, still more preferably 1 to 4, still more preferably 1 to 3, still more preferably 1 to 2, and still more preferably 1, may be exemplified.

As the nucleotide sequence of the peptide nucleic acid moiety, preferably, it consists only of a nucleotide sequence complementary to miR-96-5p, and most preferably, it consists only of a nucleotide sequence (SEQ ID NO: 1) complementary to human miR-96-5p.

In the inhibitor of the present invention, the additional moiety that is covalently linked to the peptide nucleic acid moiety is not particularly limited, as long as it does not inhibit the miR-96-5p inhibitory effect of the peptide nucleic acid moiety. For example, a peptide that can be added to the N-terminus and/or the C-terminus of the peptide nucleic acid moiety, such as octaarginine, TAT, NLS, TP10, PDEP-P14, Penetratin, Pip2b, or CLIP6, may be exemplified.

As the peptide that can be added to the N-terminus and/or the C-terminus of the peptide nucleic acid moiety, for example, a peptide consisting of 1 to several amino acids may be exemplified. The number of amino acid residues constituting the peptide (in the case of both ends, the number of amino acid residues per peptide) may be, for example, 1 to several, preferably 1 to 6, more preferably 1 to 5, still more preferably 1 to 4, still more preferably 1 to 3, and still more preferably 2 to 3. Each lower limit and each upper limit can be arbitrarily combined as desired.

The peptide that can be added to the N-terminus and/or the C-terminus of the peptide nucleic acid moiety can constitute the peptide moiety in the inhibitor of the present invention. The peptide preferably contains one or more basic amino acids, such as lysine, arginine, histidine, and tryptophan, so that the sum of the charges of the peptide moieties is positive. This is because it is speculated that if the peptide moiety is positively charged, it will interact electrostatically with the target microRNA (negatively charged), which is negatively charged by phosphate groups.

The inhibitor of the present invention can be prepared by a PNA synthesis method well known in the art, for example, by solid-phase synthesis by the Fmoc method using commercially available PNA monomers.

The inhibitor of the present invention exhibits the miR-96-5p inhibitory effect.

The term "miR-96-5p inhibitory effect" as used herein means an inhibitory effect on miR-96-5p activity.

The term "miR-96-5p activity" as used herein means activity that binds to the 3'-untranslated region (EAAC1 3'-UTR) of its target sequence, cysteine transporter excitatory amino acid carrier 1 (EAAC1), or, as a result, activity that inhibits the expression of the target EAAC1.

EAAC1 mRNA expression can be detected or quantified by various analytical methods well known in the art, such as in situ hybridization, Northern blotting, dot blot, RNase protection assay, RT-PCR, Real-Time PCR, qRT-PCT, DNA array analysis, and the like. EAAC1 protein expression can be detected or quantified by various analytical methods well known in the art, such as in situ hybridization, Western blotting, various immunohistological methods, and the like.

The binding of miR-96-5p to EAAC1 3'-UTR can be quantified by a well-known method widely used in microRNA analysis, such as the luciferase reporter assay used in the Examples described later. In the luciferase reporter assay, the binding of miR-96-5p to EAAC1 3'-UTR can be quantified by preparing a microRNA reporter plasmid in which EAAC1 3'-UTR is inserted downstream of the luciferase reporter gene, introducing this plasmid into cultured cells, and measuring the luciferase activity.

Although it is not limited to the following, the miR-96-5p inhibitory effect by the inhibitor of the present invention can be evaluated, for example, by introducing the microRNA reporter plasmid into cultured cells, and at the same time, introducing various microRNA mimics and the inhibitor of the present invention into the cultured cells.

More particularly, assuming that the luciferase activity when only the negative control mimic is introduced is 1, the luciferase activity when only the miR-96-5p mimic is introduced is reduced by the action of miR-96-5p activity. Simultaneous introduction of the miR-96-5p mimic and the inhibitor of the present invention restores the luciferase activity, which is reduced by administration of the miR-96-5p mimic alone, to 1 by the miR-96-5p inhibitory effect of the inhibitor of the present invention.

As the negative control, a miRNA (for example, cel-miR-39-3p) that has been confirmed to have no target in the human genes can be used.

### «Pharmaceutical composition of the present invention»

The pharmaceutical composition of the present invention can be prepared in a form capable of delivering the inhibitor of the present invention that is the active ingredient into the brain. For example, the naked DNA introduction method described in WO 90/11092, US Patent No. 5,580,859, or the like can be used. In the naked DNA introduction method, introduction efficiency can be improved using biodegradable latex beads. PNA-coated latex beads, which are coated with PNA instead of DNA, are efficiently transported into cells by the beads after the initiation of endocytosis. This method can be further improved by treating the beads to increase their hydrophobicity, thereby promoting endosomal disruption and PNA release into the cytoplasm.

As carriers for delivering the inhibitor of the present invention into cells, liposomes and lipids (for example, US Patent Nos. 7,001,614, 7,067,697, 7,214,384, and the like) and synthetic polymers (for example, US Patent No. 6,312,727 and the like) can also be used.

The pharmaceutical composition of the present invention can be delivered into the brain by intranasal administration. Intranasal administration is a method of administering a drug to the nasal mucosa, and it is known that there is a route through which the drug is then absorbed into the blood vessels or is directly transferred to the cerebrospinal fluid or nerve cells. The drug used in the present invention can reach the brain using this administration route.

The pharmaceutical composition of the present invention can be combined with pharmaceutically acceptable suitable carriers or diluents to be formulated in solid, semi-solid, liquid, or gaseous form, such as tablets, capsules, powders, granules, ointments, liquids, suppositories, injections, inhalants, aerosols, and the like.

It is desirable to use the most effective administration route for treatment, and examples thereof include oral administration, or parenteral administration, such as intranasal, intraoral, intratracheal, intrarectal, subcutaneous, intramuscular, and intravenous administration, and desirably, oral administration or intranasal administration can be mentioned.

Formulations suitable for oral administration include emulsions, syrups, capsules, tablets, powders, granules, and the like. Liquid preparations, such as emulsions and syrups, can be prepared using water; sugars such as such as sucrose, sorbitol, or fructose; glycols such as polyethylene glycol or propylene glycol; oils such as sesame oil, olive oil, or soybean oil; preservatives such as p-hydroxybenzoate esters; flavors such as strawberry flavor or peppermint; or the like, as excipients. Capsules, tablets, powders, granules, and the like, can be prepared using diluents such as lactose, glucose, sucrose, or mannitol; disintegrants such as starch or sodium alginate; lubricants such as magnesium stearate or talc; binders such as polyvinyl alcohol, hydroxypropyl cellulose, or gelatin; surfactants such as fatty acid esters; plasticizers such as glycerin; or the like, as excipients.

Formulations suitable for parenteral administration include injections, suppositories, sprays, and the like. Injections are prepared using carriers such as salt solutions, a glucose solution, or mixtures of both; or the like. Suppositories are prepared using carriers such as cocoa butter, hydrogenated fats, carboxylic acids, or the like. Sprays are prepared using carriers or the like that do not irritate the recipient's oral cavity and respiratory tract mucosa and that disperse the active ingredient as fine particles to facilitate absorption. Specific examples of the carriers for sprays include lactose and glycerin. Formulations, such as aerosols, dry powders, and the like, are possible depending on the properties of the inhibitor of the present invention and the carrier used. In these parenteral formulations, the ingredients exemplified as excipients for oral formulations can also be added.

The dosage or frequency of administration varies depending on the desired therapeutic effect, administration method, treatment period, age, body weight, and the like, but is usually 10 µg/kg to 20 mg/kg per day for adults. The target dosage can be set so that the concentration of the inhibitor of the present invention in blood samples drawn within the first 24 to 48 hours after administration of the drug is in the range of approximately 0.1-1000 µmol/L, approximately 0.5-500 µmol/L, approximately 1-100 µmol/L, or approximately 50-50 µmol/L.

Since the active ingredient, the inhibitor of the present invention, has miR-96-5p inhibitory activity, the pharmaceutical composition of the present invention can be used for prevention or treatment of diseases in which miR-96-5p is involved.
miR-96-5p can decrease intraneuronal concentrations of glutathione, a major antioxidant in the nervous system. Glutathione is a tripeptide consisting of cysteine, glutamic acid, and glycine. Cysteine is the rate-limiting factor for intraneuronal glutathione synthesis because the amount of glutamic acid and glycine is sufficient in neurons. Since cysteine uptake into neurons is mediated by cysteine transporter excitatory amino acid carrier 1 (EAAC1), miR-96-5p, which inhibits EAAC1 expression, reduces the concentration of intraneuronal glutathione by inhibiting EAAC1 expression.

Glutathione is an important protective molecule against cerebral oxidative stress, and in neurodegenerative diseases caused by oxidative stress in the brain, such as Alzheimer's disease, Parkinson's disease, multiple system atrophy, or amyotrophic lateral sclerosis, it has been confirmed that the intraneuronal glutathione concentration is decreased, and that miR-96-5p is increased in some neurodegenerative diseases.

Therefore, the diseases in which miR-96-5p is involved include neurodegenerative diseases caused by oxidative stress in the brain or neurodegenerative diseases in which the amount of intraneuronal glutathione is reduced. Specific examples include Alzheimer's disease, Parkinson's disease, multiple system atrophy, or amyotrophic lateral sclerosis.

### EXAMPLES

The present invention will now be further illustrated by, but is by no means limited to, the following Examples.

### «Example 1: Synthesis of antisense PNAs against human miR-96-5p»

In this Example, two types of antisense peptide nucleic acid (PNA) derivatives against human miR-96-5p consisting of the following sequences A and B were synthesized as miR-96-5p inhibitors of the present invention.

The antisense peptide nucleic acid derivative represented by sequence A consists of:
a peptide nucleic acid moiety consisting of the nucleotide sequence of SEQ ID NO: 1,
an oligopeptide moiety consisting of two amino acids (Cys-Lys) added to the N-terminal side of the peptide nucleic acid moiety, and
an oligopeptide moiety consisting of three amino acids (Lys-Lys-Lys) added to the C-terminal side of the peptide nucleic acid moiety.

The antisense peptide nucleic acid derivative represented by sequence B consists of:
the peptide nucleic acid moiety consisting of the nucleotide sequence of SEQ ID NO: 1,
an oligopeptide moiety consisting of one amino acid (Lys) added to the N-terminal side of the peptide nucleic acid moiety, and
the oligopeptide moiety consisting of three amino acids (Lys-Lys-Lys) added to the C-terminal side of the peptide nucleic acid moiety.

Sequence A: CysLys-AGCAAAAATGTGCTAGTGCCAAA-LysLysLys
Sequence B: Lys-AGCAAAAATGTGCTAGTGCCAAA-LysLysLys

Hereinafter, the antisense peptide nucleic acid (PNA) derivative is simply referred to as "antisense PNA", and the antisense PNA against human miR-96-5p is abbreviated as "PNA-miR-96".

The synthesis of PNA-miR-96 was carried out in accordance with the method described in Avitabile, C., Moggio, L., D'Andrea, L. D., Pedone, C., Romanelli, A. (2010) Development of an efficient and low-cost protocol for the manual PNA Synthesis by Fmoc chemistry. Tetrahedron Lett. 51 (29), 3716-3718.

More particularly, the solid-phase synthesis of antisense PNAs against miR-96-5p were carried out by an Fmoc method using commercially available PNA monomers. After removing Fmoc groups on the solid-phase support by piperidine treatment, amino acids and PNA monomers were sequentially coupled. The completion of each reaction was confirmed by detecting the presence of the terminal amino group with ninhydrin reaction. The final step of deprotection and cleavage from the solid-phase support was carried out with trifluoroacetic acid (TFA). The PNA oligomers of interest were analyzed and purified by HPLC, and their structures were confirmed by Mass spectrum.

### «Example 2: Examination of inhibitory effect of antisense PNAs on miR-96-5p activity by luciferase reporter assay»

In this Example, the antisense PNA against human miR-96-5p (PNA-miR-96) consisting of sequence A synthesized in Example 1 was examined for its inhibitory effect on miR-96-5p activity using luciferase reporter assay.

### (2-1) Construction of assay system

A plasmid comprising the 3'-untranslated region (EAAC1 3'-UTR) of the target sequence of human miR-96-5p, i.e., cysteine transporter excitatory amino acid carrier 1 (EAAC1), and a firefly luciferase reporter gene was constructed in accordance with the method described in Kinoshita, C. et al. Rhythmic oscillations of the microRNA miR-96-5p play a neuroprotective role by indirectly regulating glutathione levels. Nat. Commun. 5:3823 doi: 10.1038/ncomms4823 (2014).

In brief, the human EAAC1 3'-UTR was amplified from cDNAs derived from human neuroblastoma SH-SY5Y cells, and then inserted downstream of the firefly luciferase reporter gene in a firefly luciferase reporter vector pMIR-REPORT (Promega, Madison, WI) to construct the desired plasmid (hereinafter referred to as the pMIR-EAAC1 3'-UTR construct).

As a plasmid for correcting the introduction efficiency in electroporation described later, a Renilla luciferase vector pRL (Promega, Madison, WI) containing a Renilla luciferase gene was prepared.

As an miR-96-5p mimic, a double-stranded RNA (Exiqon) consisting of a guide strand having an unmodified mature miR-96-5p sequence, and two passenger strands was used. The two passenger strands were prepared by dividing a sequence complementary to the guide strand into two stands, and were LNA (locked nucleic acid) modified RNA strands.

Furthermore, as a negative control (NC), a negative control mimic (Exiqon) was prepared based on a sequence of miRNA that had been confirmed to have no target in the human genes.

### (2-2) Electroporation

The SH-SY5Y cells, the pMIR-EAAC1 3'-UTR construct (firefly luciferase) and vector pRL (Renilla), each miRNA mimic (miR-96-5p mimic, NC mimic), and each miR-96-5p inhibitor were prepared appropriately so that it could be used at 50 µL, 10 µL, 10 µL, and 30 µL per electroporation, respectively.

PNA-miR-96 was preheated at 60°C for 5 minutes.

Immediately before electroporation, each of the above components was mixed in amounts of 50 µL, 10 µL, 10 µL, and 30 µL, and electroporation was carried out under the following conditions:
Pouring pulse
voltage: 150 V, pulse width: 2 ms, pulse interval: 50 ms, pulse count: 2 times, attenuation rate: 10%, and polarity +.
Transfer pulse
voltage: 20 V, pulse width: 50 ms, pulse interval: 50 ms, pulse count: ±5 times, attenuation rate: 40%, and polarity +/-.

In connection with this, "polarity +/-" and "pulse count: ±5 times" mean that positive and negative voltages are applied alternately and repeated five times.

After electroporation, the cells were cultured in serum-containing DMEM medium for 2 days, and luciferase assay was carried out.

### (2-3) Results

The results are shown in Figure 1. Table 2 shows the combinations of mimics and the inhibitor used in each luciferase assay shown in Figure 1.

**[Table 2]**

| No. | Mimic | Inhibitor |
|---|---|---|
| 1 | Negative control (NC) | - |
| 2 | miR-96 | - |
| 3 | miR-96 | PNA-miR-96 (1 µmol/L) |

Assuming that the luciferase activity when only the negative control (NC) mimic was introduced was 1, the luciferase relative activity when only the miR-96-5p mimic was introduced was reduced to approximately 0.55 by the action of miR-96-5p activity. Simultaneous introduction of the miR-96-5p mimic and the inhibitor (PNA-miR-96) of the present invention restored the luciferase activity, which was reduced by administration of the miR-96-5p mimic alone, to approximately 1 by the miR-96-5p inhibitory effect of the inhibitor of the present invention.

### INDUSTRIAL APPLICABILITY

The present invention can be used for the production of preventive or therapeutic drugs for neurodegenerative diseases, such as Alzheimer's disease, Parkinson's disease, multiple system atrophy, amyotrophic lateral sclerosis, or the like.

### FREE TEXT IN SEQUENCE LISTING

The nucleotide sequence of SEQ ID NO: 1 in the sequence listing is an antisense PNA for miR-96-5p.

## Claims

1. An miR-96-5p inhibitor comprising a peptide nucleic acid moiety comprising a nucleotide sequence complementary to miR-96-5p.

2. The miR-96-5p inhibitor according to claim 1, wherein a peptide moiety consisting of 1 to several amino acids is added to an N-terminus and/or a C-terminus of the peptide nucleic acid moiety.

3. The miR-96-5p inhibitor according to claim 1 or 2, wherein the peptide nucleic acid moiety consists only of a nucleotide sequence complementary to miR-96-5p.

4. The miR-96-5p inhibitor according to any one of claims 1 to 3, wherein the nucleotide sequence complementary to miR-96-5p is a nucleotide sequence of SEQ ID NO: 1.

5. A pharmaceutical composition comprising the miR-96-5p inhibitor according to any one of claims 1 to 4 as an active ingredient.

6. The pharmaceutical composition according to claim 5 for prevention or treatment of a neurodegenerative disease in which a decrease in intraneuronal glutathione is observed.

7. The pharmaceutical composition according to claim 5 or 6, wherein the neurodegenerative disease is Alzheimer's disease, Parkinson's disease, multiple system atrophy, or amyotrophic lateral sclerosis.
